# EUROPEAN PATENT APPLICATION

(11) **EP 4 083 057 A1**
(43) Date of publication of application: **02.11.2022**
(21) Application number: 20905873.4
(22) Date of filing: 24.12.2020
(51) Int. Cl.: C07K 14/435, C12N 5/00, C12N 5/07, C12Q 1/02, G01N 33/48, G01N 33/53, G01N 33/543

(54) **METHOD FOR DETECTING PROTEIN IN EXTRACELLULAR VESICLES AND EXTRACELLULAR VESICLE MEMBRANE PERMEABILIZATION TREATMENT AGENT**

(30) Priority: 27.12.2019 JP 2019238075; 27.12.2019 JP 2019238071
(71) Applicant: Tosoh Corporation, Yamaguchi 746-8501 (JP)
(72) Inventor: AKIYAMA, Yasuyuki, Ayase-shi, Kanagawa 252-1123 (JP); OHTAKE, Norihisa, Ayase-shi, Kanagawa 252-1123 (JP)
(74) Representative: Novagraaf International SA
(86) International application number: PCT/JP2020/048510
(87) International publication number: WO 2021/132489

(57) **Abstract**

The present invention aims to provide a method capable of simply and accurately detecting a particular protein contained in an extracellular vesicle, and a membrane permeabilization treatment agent that can be used for the method. The object can be achieved by a method of detecting a particular protein contained in an extracellular vesicle, the method including the steps of: (A) capturing the extracellular vesicle using a carrier capable of binding to an extracellular-vesicle-specific marker present on the surface of the extracellular vesicle; (B) carrying out membrane permeabilization treatment for the extracellular vesicle captured by the carrier, using a membrane permeabilization treatment agent; and (C) introducing a reagent capable of detecting the particular protein contained in the extracellular vesicle, into the membrane-permeabilized extracellular vesicle; wherein the Step (B) is carried out such that the particular protein does not leak to the outside of the extracellular vesicle, and such that the reagent can be introduced into the extracellular vesicle.

## Description

### TECHNICAL FIELD

The present invention relates to a method capable of simply and accurately detecting a particular protein contained in an extracellular vesicle, and a membrane permeabilization treatment agent that can be used for the method.

### BACKGROUND ART

It is known that body fluids such as blood, and cell culture liquids and the like contain extracellular vesicles secreted from cells contained in the body fluids and the culture liquids. It has recently been reported that the extracellular vesicles have a function as a mediator of intercellular communication in the living body, and that they are involved in diseases such as cancer and in physiological phenomena. Thus, studies are in progress aiming at elucidation of their physiological functions, and their application to examination for diseases.

An extracellular vesicle, represented by an exosome and an apoptotic vesicle, is a colloidal particle coated with a lipid bilayer membrane. A detection method for an extracellular vesicle, wherein a protein on the membrane surface is targeted, has so far been reported (Patent Document 1).

As a detection method for a particular protein contained in an extracellular vesicle, a method in which the membrane is permeabilized with a surfactant containing sodium deoxycholate and lauroyl sarcosinate, and then protein that leaked from the inside of the extracellular vesicle is detected using a mass spectrometer, has been reported (Patent Document 2). Further, a method in which extracellular vesicles are concentrated using magnetic particles, and then the membrane of the extracellular vesicles are solubilized, followed by detecting a particular protein that leaked from the inside of the extracellular vesicles by Western blotting, has been reported (Patent Document 3). However, the detection methods described in these documents are laborious, and moreover, since these methods carry out the detection after the leakage of the particular protein to the outside of the extracellular vesicle, accurate detection by the methods is difficult.

### PRIOR ART DOCUMENTS

### [Patent Documents]

[Patent Document 1] Japanese Translated PCT Patent Application Laid-open No. 2012-508577
[Patent Document 2] WO 2015/182580
[Patent Document 3] WO 2016/186215

### SUMMARY OF THE INVENTION

### PROBLEMS TO BE SOLVED BY THE INVENTION

An object of the present invention is to provide a method capable of simply and accurately detecting a particular protein contained in an extracellular vesicle, and a membrane permeabilization treatment agent that can be used for the method.

### MEANS FOR SOLVING THE PROBLEMS

In order to solve the above problem, the present inventors intensively studied to discover that use of a particular membrane permeabilization treatment agent enables introduction of a reagent capable of detecting a particular protein into an extracellular vesicle without allowing leakage of the particular protein contained in the extracellular vesicle to the outside of the extracellular vesicle, thereby reaching the present invention.

More specifically, the present invention can be exemplified as follows.
[1] A method of detecting a particular protein contained in an extracellular vesicle, the method comprising the steps of:
   (A) capturing the extracellular vesicle using a carrier capable of binding to an extracellular-vesicle-specific marker present on the surface of the extracellular vesicle;
   (B) carrying out membrane permeabilization treatment for the extracellular vesicle captured by the carrier, using a membrane permeabilization treatment agent; and
   (C) introducing a reagent capable of detecting the particular protein contained in the extracellular vesicle, into the membrane-permeabilized extracellular vesicle, and
   wherein the Step (B) is carried out such that the particular protein does not leak to the outside of the extracellular vesicle, and such that the reagent can be introduced into the extracellular vesicle.
[2] The method, further comprising a step of removing an impurity after the Step (A) and/or after the Step (B).
[3] The method, further comprising a step of detecting the particular protein
   using the introduced reagent, after the Step (C).
[4] The method, wherein the membrane permeabilization treatment agent is a surfactant and/or an organic solvent.
[5] The method, wherein the surfactant is an anionic surfactant, a cationic surfactant, or a nonionic surfactant.
[6] The method, wherein the surfactant is deoxycholate, glycocholate, SDS, saponin, Triton X-100, or CTAB.
[7] The method, wherein the surfactant is any of the following (a) to (f):
   (a) 0.05 to 0.5% (w/v) deoxycholate
   (b) 0.01 to 1% (w/v) glycocholate
   (c) 0.01 to 0.1% (w/v) SDS
   (d) 0.1 to 2% (w/v) saponin
   (e) 0.005 to 0.5% (w/v) Triton X-100
   (f) 0.002 to 0.2% (w/v) CTAB.
[8] The method, wherein the surfactant is any of the following (a) to (c):
   (a) 0.05 to 0.5% (w/v) deoxycholate
   (b) 0.01 to 1% (w/v) glycocholate
   (c) 0.01 to 0.1% (w/v) SDS.
[9] The method, wherein the organic solvent is ethanol and/or acetone.
[10] The method, wherein the organic solvent is the following (a) or (b):
   (a) 20 to 60% (v/v) ethanol
   (b) 20 to 70% (v/v) acetone.
[11] The method, wherein the carrier is a magnetic particle.
[12] A membrane permeabilization treatment agent for an extracellular vesicle,
   the agent comprising a surfactant and/or an organic solvent,
   the agent being capable of allowing membrane permeabilization treatment of the extracellular vesicle such that a particular protein contained in the extracellular vesicle does not leak to the outside of the extracellular vesicle, and such that a reagent capable of detecting the particular protein contained in the extracellular vesicle can be introduced into the extracellular vesicle.
[13] The membrane permeabilization treatment agent, wherein the surfactant is an anionic surfactant, a cationic surfactant, or a nonionic surfactant.
[14] The membrane permeabilization treatment agent, wherein the surfactant is one or more components selected from the group consisting of deoxycholate, glycocholate, SDS, saponin, Triton X-100, and CTAB.
[15] The membrane permeabilization treatment agent, wherein the surfactant is any of the following (a) to (f):
   (a) 0.05 to 0.5% (w/v) deoxycholate
   (b) 0.01 to 1% (w/v) glycocholate
   (c) 0.01 to 0.1% (w/v) SDS
   (d) 0.1 to 2% (w/v) saponin
   (e) 0.005 to 0.5% (w/v) Triton X-100
   (f) 0.002 to 0.2% (w/v) CTAB.
[16] The membrane permeabilization treatment agent, wherein the surfactant is any of the following (a) to (c):
   (a) 0.05 to 0.5% (w/v) deoxycholate
   (b) 0.01 to 1% (w/v) glycocholate
   (c) 0.01 to 0.1% (w/v) SDS.
[17] The membrane permeabilization treatment agent, wherein the organic solvent is ethanol and/or acetone.
[18] The membrane permeabilization treatment agent, wherein the organic solvent is the following (a) or (b):
   (a) 20 to 60% (v/v) ethanol
   (b) 20 to 70% (v/v) acetone.
[19] A kit for detecting a particular protein contained in an extracellular vesicle, the kit comprising:
   a magnetic particle capable of binding to an extracellular-vesicle-specific marker present on the surface of the extracellular vesicle;
   the membrane permeabilization treatment agent; and
   a reagent capable of detecting the particular protein contained in the extracellular vesicle.

### BRIEF DESCRIPTION OF THE DRAWINGS

Fig. 1 is a diagram illustrating an example of the method of the present invention.
Fig. 2 is a diagram (photograph) showing the result of Western blotting in Comparative Example 8.

### MODE FOR CARRYING OUT THE INVENTION

The present invention is described below in detail.

### <1> Method of Present Invention

The method of the present invention is a method of detecting a particular protein contained in an extracellular vesicle.

The method of the present invention may include the steps of:
carrying out membrane permeabilization treatment for an extracellular vesicle using a membrane permeabilization treatment agent (which may hereinafter be simply referred to as "membrane permeabilization step"); and
introducing a reagent capable of detecting the particular protein contained in the extracellular vesicle, into the membrane-permeabilized extracellular vesicle (which may hereinafter be simply referred to as "introduction step").

The extracellular-vesicle-specific marker present on the surface of an extracellular vesicle may hereinafter be simply referred to as "vesicle-specific marker". The reagent capable of detecting the particular protein may hereinafter be simply referred to as "detection reagent".

"Extracellular vesicle" means a lipid-coated vesicle having a diameter of 1 nm to 1 µm, actively or passively released from a cell. Examples of the extracellular vesicle include exosomes, microvesicles, ectosomes, membrane particles, exosome-like vesicles, and apoptotic vesicles (Nature Reviews, 9, 2009, 581-593). It has been reported that, in general, an extracellular vesicle is constituted by lipids and proteins having a composition different from those of cells (BioScience, 65, 2015, 783-797).

The origin of the extracellular vesicle is not limited. Examples of the origin of the extracellular vesicle include: body fluids; bacterial suspensions; culture liquids and culture supernatants after cell culture; and homogenates of tissue cells. The origin of the extracellular vesicle is especially preferably a body fluid or a culture supernatant after cell culture. Examples of the body fluid include components in the blood composition, such as whole blood, serum, plasma, blood components, various blood cells, blood clots, and platelets; urine; semen; breast milk; sweat; interstitial fluid; interstitial lymph fluid; bone marrow fluid; tissue fluid; saliva; gastric juice; joint fluid; pleural effusion; bile; ascites; and amniotic fluid. The body fluid is especially preferably a component in the blood composition. The body fluid such as a component in the blood composition may be a body fluid treated with an anticoagulant such as citric acid, heparin, or EDTA.

The method of the present invention may include a step of capturing an extracellular vesicle (which may hereinafter be simply referred to as "capturing step") before the membrane permeabilization step. The term "capturing of an extracellular vesicle" may be interchangeably used with the term "collecting of an extracellular vesicle". The capturing step may be carried out, for example, using a carrier capable of binding to an extracellular-vesicle-specific marker present on the surface of the extracellular vesicle. Thus, the extracellular vesicle to be subjected to the membrane permeabilization step may be, for example, an extracellular vesicle captured on the carrier.

Thus, more specifically, the method of the present invention may include, for example, the steps of:
capturing an extracellular vesicle using a carrier capable of binding to an extracellular-vesicle-specific marker present on the surface of the extracellular vesicle;
carrying out membrane permeabilization of the extracellular vesicle captured by the carrier, using a membrane permeabilization treatment agent; and
introducing a reagent capable of detecting the particular protein contained in the extracellular vesicle, into the membrane-permeabilized extracellular vesicle.

The carrier used in the capturing step can be obtained by, for example, immobilizing a substance capable of specifically binding to the vesicle-specific marker, on a base material.

The base material is not limited as long as it is a water-insoluble substance. The base material may be either hydrophilic or hydrophobic. As the base material, known materials commonly used in the art may be used without limitation. Specific examples of such materials include polysaccharides such as agarose-based polysaccharides, dextran-based polysaccharides, chitosan-based polysaccharides, and cellulose-based polysaccharides; synthetic organic polymers such as polyacrylamide-based polymers, polyvinyl alcohol-based polymers, polyvinylpyrrolidone-based polymers, polyacrylonitrile-based polymers, styrene-divinylbenzene copolymers, polystyrene-based polymers, acrylate-based polymers, methacrylate-based polymers, polyethylene-based polymers, polypropylene-based polymers, polytetrafluoroethylene-based polymers, ethylene-vinyl acetate copolymer-based polymers, polyamide-based polymers, polycarbonate-based polymers, polyvinylidene fluoride-based polymers, polyvinyl formal-based polymers, polyarylate-based polymers, and polyether sulfone-based polymers; inorganic substances such as glass-based substances, titanium-based substances, activated carbon-based substances, ceramic-based substances including alumina, silica, and hydroxyapatite, and metal-based substances including iron oxide and gold. The shape of the base material is not limited. Specific examples of the shape of the base material that may be used include known shapes such as a particle shape, fibrous shape, hollow fiber shape, film shape, and flat plate shape. In particular, the shape of the base material is preferably a particle shape since it has a large surface area; it can homogeneously and efficiently bind to a target cell; it is less-likely to cause physical damage to a cell; it is less-likely to be damaged; and a uniform carrier can be easily obtained therefrom. The shape of the particle is not limited. The particle is preferably a spherical particle (including not only perfectly spherical particles, but also almost spherical particles) since it can be easily handled; it is less likely to cause physical damage to an extracellular vesicle; it is less likely to be damaged; and a uniform particle can be easily obtained. Further, the particle is especially preferably a magnetic particle since it can be simply, quickly, and accurately collected by application of an external magnetic field. Examples of the magnetic particle include particles produced such that they contain a magnetic body.

The vesicle-specific marker is not limited as long as it is a substance specifically present on the surface of an extracellular vesicle. Examples of the vesicle-specific marker include antigenic substances present on the surface of an extracellular vesicle, and substances recognized by a particular receptor, present on the surface of an extracellular vesicle. Specific examples of the vesicle-specific marker include proteins, sugar chains, nucleic acids, and lipids. For example, in cases where the extracellular vesicle is an exosome, examples of the antigenic substances present on the surface of an extracellular vesicle include tetraspanins such as CD9, CD63, and CD81; antigen presentation-related proteins such as MHC (Major Histocompatibility Complex) I and MHC II; adhesion molecules such as integrin, ICAM-1 (InterCellular Adhesion Molecule 1), and EpCAM (Epithelial Cell Adhesion Molecule); cytokines and cytokine receptors such as EGFR (Epidermal Growth Factor Receptor) vIII and TGF (Transforming Growth Factor)-β; and enzymes. Further, for example, in cases where the extracellular vesicle is an exosome, examples of the substances recognized by a particular receptor, present on the surface of an extracellular vesicle include lipids such as phosphatidylserine.

Examples of the substance capable of specifically binding to the vesicle-specific marker include antibodies against a protein specifically present on the surface of an extracellular vesicle, lectins capable of specifically binding to a sugar chain specifically present on the surface of an extracellular vesicle, aptamers capable of specifically binding to a protein or nucleic acid specifically present on the surface of an extracellular vesicle, and receptors of a lipid specifically present on the surface of an extracellular vesicle. The substance capable of specifically binding to the vesicle-specific marker is especially preferably an antibody against a protein specifically present on the surface of an extracellular vesicle. In cases where a substance capable of specifically binding to a membrane surface marker derived from a particular organ or a particular cell from which an extracellular vesicle is released is used as the substance capable of specifically binding to the vesicle-specific marker, the extracellular vesicle released from the particular organ or the particular cell can be specifically captured. Examples of the method of immobilizing the substance capable of specifically binding to the vesicle-specific marker, on the base material include covalent bonding, electrostatic interaction, hydrophobic interaction, and coordinate bonding. The method of immobilizing the substance capable of specifically binding to the vesicle-specific marker, on the base material is especially preferably covalent bonding, which is a strong modification method.

The membrane permeabilization step is a step of carrying out membrane permeabilization treatment of an extracellular vesicle using a membrane permeabilization treatment agent. The "membrane permeabilization treatment of an extracellular vesicle" may mean treatment for increasing membrane permeability of the extracellular vesicle. More specifically, it may mean treatment for increasing membrane permeability of the extracellular vesicle to the detection reagent.

The membrane permeabilization step is carried out such that the particular protein contained in the extracellular vesicle does not leak to the outside of the extracellular vesicle, and such that the detection reagent can be introduced into the extracellular vesicle. More specifically, the membrane permeabilization step may be carried out to form membrane-penetrating pores having a size which does not allow leakage of the particular protein contained in the extracellular vesicle to the outside of the extracellular vesicle, but which allows introduction of the detection reagent into the extracellular vesicle. In cases where the size of the membrane-penetrating pores is small, the detection reagent cannot enter into the extracellular vesicle, while in cases where the size of the membrane-penetrating pores is large, leakage of proteins present in the extracellular vesicles occur. Thus, in either case, accurate detection of a protein present in the extracellular vesicle is difficult. The term "does not allow leakage of the particular protein to the outside of the extracellular vesicle" means that the remaining amount, in the vesicle, of the particular protein contained in the extracellular vesicle is sufficient for its accurate detection by the detection reagent. More specifically, the term "does not allow leakage of the particular protein to the outside of the extracellular vesicle" may mean, for example, that the amount (such as the number of molecules) of the particular protein remaining in the extracellular vesicle after carrying out the membrane permeabilization step is not less than 20%, not less than 30%, not less than 40%, not less than 50%, not less than 60%, not less than 70%, not less than 80%, or not less than 90% with respect to the amount (such as the number of molecules) of the particular protein contained in the extracellular vesicle before carrying out the membrane permeabilization step.

The membrane permeabilization treatment agent is not limited as long as the membrane permeabilization step can be carried out such that the particular protein contained in the extracellular vesicle does not leak to the outside of the extracellular vesicle, and such that the detection reagent can be introduced into the extracellular vesicle. More specifically, the membrane permeabilization treatment agent may be capable of forming membrane-penetrating pores having a size which does not allow leakage of the particular protein contained in the extracellular vesicle to the outside of the vesicle, but which allows introduction of the detection reagent into the vesicle. Either a single component, or a combination of two or more components may be used as the membrane permeabilization treatment agent.

Examples of the membrane permeabilization treatment agent include surfactants and organic solvents. The membrane permeabilization treatment agent is preferably a surfactant. As the membrane permeabilization treatment agent, at least a surfactant may be preferably used. A combination of a surfactant and an organic solvent may be more preferably used.

Examples of the surfactant include cationic surfactants, nonionic surfactants, and anionic surfactants. Examples of the cationic surfactants include didodecyldimethylammonium bromide (DDAB), cetyltrimethylammonium bromide (CTAB), cetylpyridinium bromide (CPB), dodecyltrimethylammonium chloride (DOTAC), sodium perfluorononanoate (SPFN), and hexadecyltrimethylammonium bromide (HDTMA). Examples of the nonionic surfactants include Triton X-100 (trade name) and saponin. Examples of the anionic surfactants include alkyl sulfuric acid ester salts (including SDS (sodium dodecyl sulfate)), bile acid and bile acid salts (including cholates such as sodium collate; deoxycholates such as sodium deoxycholate; glycocholates such as sodium glycocholate; chenodeoxycholates such as sodium chenodeoxycholate; lithocholates such as sodium lithocholate; glycolithocholates such as sodium glycolithocholates; and taurolithocholates such as sodium taurolithocholate), sodium lauryl sulfosuccinate, sodium α-olefin sulfonate, sodium polyoxyethylene lauryl ether sulfate, sodium polyoxyethylene lauryl ether phosphate, and sodium laurylbenzene sulfonate. The surfactant is especially preferably an anionic surfactant from the viewpoint of accurately detecting the particular protein in the extracellular vesicle. The anionic surfactant is especially preferably bile acid or a bile acid salt since they do not have protein-denaturing properties. Preferred examples of the surfactant include deoxycholates (such as sodium deoxycholate), glycocholates (such as sodium glycocholate), SDS, saponin, Triton X-100, and CTAB. More preferred examples of the surfactant include deoxycholates (such as sodium deoxycholate), glycocholates (such as sodium glycocholate), and SDS.

Examples of the organic solvent include alcohols such as ethanol and methanol; and acetone. Preferred examples of the organic solvent include ethanol and acetone.

The concentration of the membrane permeabilization treatment agent may be appropriately set depending on conditions such as the type of the membrane permeabilization treatment agent and the amount of the extracellular vesicle to be subjected to the membrane permeabilization treatment. The membrane permeabilization treatment agent may be used in the form of, for example, an aqueous solution containing a predetermined concentration of the membrane permeabilization treatment agent. The aqueous solution containing the membrane permeabilization treatment agent may be prepared by, for example, diluting the membrane permeabilization treatment agent in water or an aqueous medium such as an aqueous buffer. Unless otherwise specified, the "concentration of the membrane permeabilization treatment agent" may mean the concentration of the membrane permeabilization treatment agent in contact with the extracellular vesicle.

In cases where a surfactant is used as the membrane permeabilization treatment agent, the concentration of the surfactant may be appropriately set depending on conditions such as the critical micelle concentration of the surfactant and the amount of the extracellular vesicle to be subjected to the membrane permeabilization treatment. The concentration of the surfactant may be, for example, not less than 0.002% (w/v), not less than 0.005% (w/v), not less than 0.01% (w/v), not less than 0.02% (w/v), not less than 0.05% (w/v), or not less than 0.1% (w/v), or may be, for example, not more than 2% (w/v), not more than 1% (w/v), not more than 0.5% (w/v), not more than 0.2% (w/v), or not more than 0.1% (w/v), or may be a consistent combination thereof.

Preferred examples of the concentration of the surfactant include the following (a) to (f). Thus, the surfactant may be, for example, any of the following (a) to (f). More specifically, for example, the surfactant may be an aqueous solution containing any of the following (a) to (f):
(a) 0.05 to 0.5% (w/v) deoxycholate
(b) 0.01 to 1% (w/v) glycocholate
(c) 0.01 to 0.1% (w/v) SDS
(d) 0.1 to 2% (w/v) saponin
(e) 0.005 to 0.5% (w/v) Triton X-100
(f) 0.002 to 0.2% (w/v) CTAB.

More preferred examples of the concentration of the surfactant include the following (a) to (c). Thus, the surfactant may be, for example, any of the following (a) to (c). More specifically, for example, the surfactant may be an aqueous solution containing any of the following (a) to (c):
(a) 0.05 to 0.5% (w/v) deoxycholate
(b) 0.01 to 1% (w/v) glycocholate
(c) 0.01 to 0.1% (w/v) SDS.

In cases where an organic solvent is used as the membrane permeabilization treatment agent, and particles are used as the carrier, the organic solvent may decrease dispersion stability in the particle suspension. When aggregation of the particles occurs due to the decrease in the dispersion stability, the capacity to detect the particular protein contained in the extracellular vesicle largely decreases. On the other hand, in cases where the concentration of the organic solvent added is low, the membrane permeabilization effect cannot be sufficiently produced, so that the capacity to detect the particular protein contained in the extracellular vesicle cannot be improved. Thus, in cases where an organic solvent is used as the membrane permeabilization treatment agent, it is preferred to use the organic solvent at a concentration which does not cause the particle aggregation, but which allows production of the membrane permeabilization effect. The concentration of the organic solvent may be, for example, not less than 5% (v/v), not less than 10% (v/v), not less than 20% (v/v), or not less than 30% (v/v), or may be, for example, not more than 90% (v/v), not more than 80% (v/v), not more than 70% (v/v), not more than 60% (v/v), or not more than 50% (v/v), or may be a combination thereof.

In cases where ethanol is used as the organic solvent, the concentration of the ethanol may be, for example, not less than 5% (v/v), not less than 10% (v/v), not less than 20% (v/v), or not less than 30% (v/v), or may be, for example, not more than 70% (v/v), not more than 60% (v/v), or not more than 50% (v/v), or may be a combination thereof. More specifically, the concentration of the ethanol is, for example, preferably 5 to 70% (v/v), more preferably 20 to 60% (v/v), still more preferably 30 to 50% (v/v). In cases where acetone is used as the organic solvent, the concentration of the acetone may be, for example, not less than 5% (v/v), not less than 10% (v/v), not less than 20% (v/v), or not less than 30% (v/v), or may be, for example, not more than 90% (v/v), not more than 80% (v/v), or not more than 70% (v/v), or may be a combination thereof. More specifically, the concentration of the acetone is, for example, preferably 5 to 80% (v/v), more preferably 20 to 70% (v/v), still more preferably 30 to 70% (v/v).

Preferred examples of the concentration of the organic solvent include the following (a) and (b). Thus, the organic solvent may be, for example, the following (a) or (b). More specifically, for example, the organic solvent may be an aqueous solution containing the following (a) or (b):
(a) 20 to 60% (v/v) ethanol
(b) 20 to 70% (v/v) acetone.

The method of the present invention may further include a step of fixing an extracellular vesicle (which may hereinafter be simply referred to as "fixation step"). The "fixation of an extracellular vesicle" may mean, for example, fixation of a particular protein contained in the extracellular vesicle. The fixation step may be carried out using a reagent for fixing an extracellular vesicle (which may hereinafter be simply referred to as "fixation treatment agent"). Examples of the fixation treatment agent include the organic solvents described above; formaldehyde; formaldehyde donor compounds (compounds capable of releasing formaldehyde by undergoing hydrolysis) such as imidazolidinyl urea; and aldehydes such as glutaraldehyde.

In cases where the method of the present invention includes the fixation step, the fixation step may be carried out, for example, at the same time as the membrane permeabilization step. Examples of cases where the fixation step and the membrane permeabilization step are carried out at the same time include a mode in which a combination of a surfactant and an organic solvent is allowed to act on the extracellular vesicle. In other words, by using a surfactant and an organic solvent in combination as the membrane permeabilization treatment agent, the fixation step and the membrane permeabilization step can be carried out at the same time. More specifically, the surfactant and the organic solvent may be used in the form of a mixture containing the surfactant and the organic solvent. The concentrations of the surfactant and the organic solvent may be those exemplified above. The surfactant can function as a membrane permeabilization treatment agent. However, in cases where the membrane permeabilization step is carried out using a surfactant alone, use of a high concentration of the surfactant may cause leakage of the particular protein contained in the extracellular vesicle, leading to difficulty in accurate detection of the particular protein. The organic solvent may have not only a function as a membrane permeabilization treatment agent, but also a function as a fixation treatment agent that makes the protein insoluble. However, in cases where the fixation step and the membrane permeabilization step are carried out using an organic solvent alone, use of a high concentration of the organic solvent may cause aggregation of the particles used as the carrier, leading to difficulty in accurate detection of the particular protein. Furthermore, in cases where the fixation step and the membrane permeabilization step are carried out using an organic solvent alone, use of a low concentration of the organic solvent may lead to insufficiency of the membrane permeabilization step, leading to difficulty in accurate detection of the particular protein. On the other hand, in cases where a mixture of a surfactant and an organic solvent is allowed to act on the extracellular vesicle, the function of the organic solvent as an fixation treatment agent causes insolubilization of the particular protein contained in the extracellular vesicle, so that the leakage of the particular protein can be suppressed, and moreover, the functions of the organic solvent and the surfactant as membrane permeabilization treatment agents promote membrane permeabilization treatment of the extracellular vesicle.

The method of the present invention may further include a step of antigen activation treatment (which may hereinafter be simply referred to as "activation step"). The "antigen activation treatment" may mean a treatment for recovering antigenicity of the particular protein. The antigenicity of the particular protein may decrease due to, for example, steric hindrance or masking of the antigenic determinant. The steric hindrance or masking of the antigenic determinant may occur when, for example, the fixation step is carried out. The decrease in the antigenicity of the particular protein may lead to a decrease in the binding performance between the particular protein and the detection reagent. Thus, by carrying out the activation step, for example, the binding performance between the particular protein and the detection reagent, which has once decreased, can be recovered, thereby allowing highly sensitive detection of the particular protein by the detection reagent. The activation step may be carried out before carrying out the introduction step. The activation step may be carried out, for example, after carrying out the fixation step. The activation step may be carried out, for example, in cases where the fixation step is not carried out. Examples of the method of the activation treatment include the heat treatment method and the protease treatment method. The method may be appropriately selected depending on conditions such as the type of the antigen. Examples of the heat treatment method include heating methods using microwave radiation, an autoclave, a thermostat bath, an electric pressure cooker, or the like. Examples of the protease treatment method include treatment by an enzyme capable of cleaving amino acids of protein, such as pepsin, trypsin, chymotrypsin, or proteinase K.

The method of the present invention may further include a step of removing an impurity (which may hereinafter be simply referred to as "removing step"). The "impurity" may mean substances that are not captured on the carrier. Examples of the impurity include substances other than an extracellular vesicle. Examples of the impurity may also include an extracellular vesicle that are not captured on the carrier. The removing step may be carried out, for example, after the capturing step, and/or after the membrane permeabilization step. In cases where the removing step is carried out, the concentrated fraction of an extracellular vesicle captured on the carrier can be subjected to the introduction step and the later-described detection step in the state where the impurity are removed, which is preferred. The removal of the impurity may be carried out, for example, based on a difference in the physical or chemical properties between the carrier and the impurity. Examples of the removal based on a difference in the physical or chemical properties include removal based on the difference in the specific gravity, difference in the sedimentation rate, difference in the particle size, difference in the dielectric constant, or the like. In cases where a magnetic particle is used as the carrier, the carrier (magnetic particle) on which an extracellular vesicle is bound can be simply, quickly, and accurately separated from an impurity by application of an external magnetic field, so that the removing step can be simply, quickly, and accurately carried out.

The introduction step is a step of introducing the detection reagent to the membrane-permeabilized extracellular vesicle. The detection reagent may be introduced into the extracellular vesicle, for example, through membrane-penetrating pores formed by carrying out the membrane permeabilization step. The detection reagent is not limited as long as the particular protein contained in the extracellular vesicle can be detected therewith.

The particular protein is not limited as long as it is a protein present in the extracellular vesicle (in other words, inside of the lipid bilayer membrane constituting the extracellular vesicle). Examples the particular protein include internal proteins common to exosomes derived from many kinds of cells, such as actin, tubulin, and GAPDH (GlycerAldehyde-3-Phosphate DeHydrogenase); sorting proteins of endosomes, such as ESCRT (Endosomal Sorting Complex Required for Transport)-III, ALIX (Apoptosis-Linked gene 2-Interacting protein X), Syntenin, and Tsg101; heat shock proteins such as HSP70 and HSP90; and proteins involved in membrane transport and fusion, such as RAB and Annexin.

The particular protein may be a protein contained in an extracellular vesicle, which protein is expressed specifically in a particular organ or a particular cell from which the extracellular vesicle is released. By detecting the protein as the particular protein, an extracellular vesicle secreted from the particular organ or the particular cell can be accurately detected. Further, by carrying out the capturing step using a carrier on which a substance capable of specifically binding to a membrane surface marker derived from a particular organ or a particular cell from which an extracellular vesicle is released is immobilized, the extracellular vesicle released from the particular organ or the particular cell can be more accurately detected.

Examples of the reagent introduced in the introduction step include antibodies and aptamers capable of specifically binding to the particular protein. The antibodies and aptamers may be modified with a labeling substance as appropriate. The labeling substance may be a substance for direct detection, or may be a substance for indirect detection. Examples of the labeling substance include dyes, enzymes, and radioactive substances. Examples of the dyes include fluorescent substances such as fluorescein isothiocyanate (FITC), phycoerythrin (PE), and Alexa Fluor (trade name). Examples of the enzymes include alkaline phosphatase and horseradish peroxidase. For example, a dye such as a fluorescent dye may be used as the labeling substance, to carry out direct detection based on the dye. It is preferred, however, to carry out the detection by using an enzyme as the labeling substance and adding a substrate that reacts with the enzyme to cause color development, from the viewpoint of highly sensitive detection of the particular protein. The mode of binding of the antibody or aptamer to the labeling substance is not limited. The antibody or aptamer may or may not be directly bound to the labeling substance. For example, the antibody may be directly bound to the labeling substance by chemical bonding or the like, or may be indirectly bound to the labeling substance by binding to a secondary antibody to which the labeling substance is bound (labeled secondary antibody). Further, for example, in cases where the antibody is modified with biotin, an enzyme modified with avidin may be used to indirectly label the antibody.

By using the introduced detection reagent, the particular protein can be detected. Thus, the method of the present invention may further include a step of detecting the particular protein using the introduced detection reagent (which may hereinafter be simply referred to as "detection step").

The detection may be carried out by an ordinary method. The detection method may be appropriately selected depending on conditions such as the type of the detection reagent. In cases where the detection reagent is an antibody capable of specifically binding to the particular protein, examples of the detection method include immunoassays such as enzyme-linked immunosorbent assay (EIA), enzyme immunometric assay (ELISA), fluorescence immunoassay (FIA), radioimmunoassay (RIA), and luminescence immunoassay. The detection method is preferably the ELISA method from the viewpoint of simple and highly sensitive detection of the antibody. Examples of the ELISA method include the competitive method and the sandwich method.

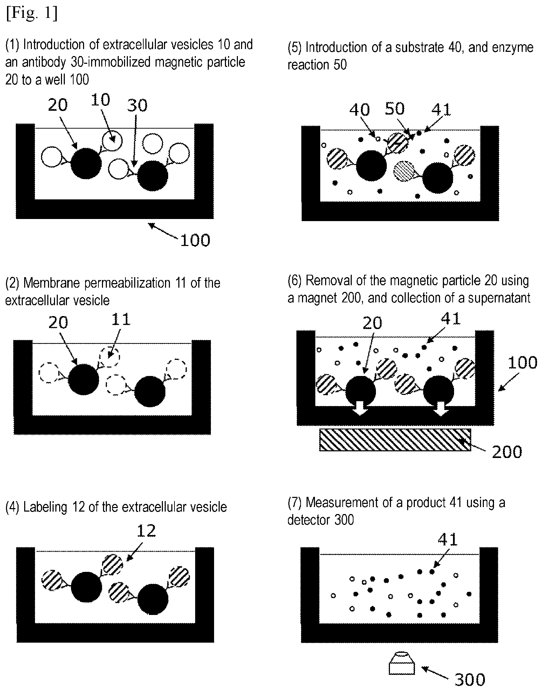

An example of the method of the present invention for a case where the detection step is carried out by the sandwich ELISA method is described below using Fig. 1.
(1) A suspension containing extracellular vesicles 10, and a magnetic particle 20 on which an antibody 30 against a vesicle-specific marker present on the membrane surface of an extracellular vesicle 10 is immobilized, is placed in a well 100 of a 96-well plate, to allow the magnetic particle 20 to capture an extracellular vesicle 10 (capturing step).
(2) After removing an extracellular vesicle 10 not captured on the magnetic particle 20, the extracellular vesicle captured on the particle surface are subjected to membrane permeabilization treatment using a membrane permeabilization treatment agent (a membrane-permeabilized extracellular vesicle 11) (membrane permeabilization step).
(3) A blocking reagent is added to the membrane-permeabilized extracellular vesicle 11 (not shown in the figure).
(4) After removing the blocking reagent, a detection reagent containing an enzyme-labeled antibody capable of specifically binding to the particular protein contained in the extracellular vesicle is introduced into the membrane-permeabilized extracellular vesicle (a labeled extracellular vesicle 12) (introduction step). While the membrane permeabilization step (2) allows the introduction of the enzyme-labeled antibody into the membrane-permeabilized extracellular vesicle, the particular protein contained in the extracellular vesicle does not leak, so that a sufficient amount of the enzyme-labeled antibody can be bound to the particular protein. Further, the blocking reagent added in (3) suppresses non-specific adsorption by the enzyme-labeled antibody and the like.
(5) A substrate 40 is added to the labeled extracellular vesicle 12. By reaction 50 of the substrate 40 with the enzyme with which the antibody bound to the particular protein contained in the extracellular vesicle is modified, an optically detectable product 41 is produced. For example, in cases where peroxidase is used as the labeling enzyme, TMB (tetramethylbenzidine) or the like may be used as the substrate 40.
(6) After adding a reagent for stopping the enzyme reaction, a magnet 200 is placed close to the bottom of the well 100, to attract the magnetic particle 20 to the well bottom, and then a solution (supernatant) containing the product 41 is collected such that the solution does not substantially contain the magnetic particle 20.
(7) The solution collected in (6) is placed in a well 100 containing no magnetic particles, and then the absorbance of the product 41 is measured by a detector 300, to detect the amount of the particular protein contained in the extracellular vesicle (detection step).

### <2> Membrane Permeabilization Treatment Agent of Present Invention

The membrane permeabilization treatment agent of the present invention is a membrane permeabilization treatment agent characterized in that the agent is capable of allowing membrane permeabilization treatment of an extracellular vesicle such that a particular protein contained in the extracellular vesicle does not leak to the outside of the extracellular vesicle, and such that a detection reagent can be introduced into the extracellular vesicle.

The membrane permeabilization treatment agent of the present invention contains a component that functions as a membrane permeabilization treatment agent (which may hereinafter be simply referred to as "membrane permeabilization treatment component"). The membrane permeabilization treatment agent of the present invention may consist of the membrane permeabilization treatment component, or may contain another component in addition to the membrane permeabilization treatment component. The membrane permeabilization treatment agent of the present invention may contain a single kind of membrane permeabilization treatment component, or may contain two or more kinds of membrane permeabilization treatment components.

To each membrane permeabilization treatment component contained in the membrane permeabilization treatment agent of the present invention, descriptions on the membrane permeabilization treatment agent in the method of the present invention may be applied (in terms of the type and the concentration of the component).

Thus, the membrane permeabilization treatment agent of the present invention may contain, for example, a surfactant and/or an organic solvent. The membrane permeabilization treatment agent of the present invention may preferably contain at least a surfactant. The agent may more preferably contain a surfactant and an organic solvent. The surfactant is preferably an anionic surfactant. Preferred examples of the surfactant include deoxycholates (such as sodium deoxycholate), glycocholates (such as sodium glycocholate), SDS, saponin, Triton X-100, and CTAB. More preferred examples of the surfactant include deoxycholates (such as sodium deoxycholate), glycocholates (such as sodium glycocholate), and SDS. Preferred examples of the organic solvent include ethanol and acetone.

The surfactant contained in the membrane permeabilization treatment agent of the present invention may be, for example, any of the following (a) to (f). More specifically, for example, the surfactant contained in the membrane permeabilization treatment agent of the present invention may be an aqueous solution containing any of the following (a) to (f):
(a) 0.05 to 0.5% (w/v) deoxycholate
(b) 0.01 to 1% (w/v) glycocholate
(c) 0.01 to 0.1% (w/v) SDS
(d) 0.1 to 2% (w/v) saponin
(e) 0.005 to 0.5% (w/v) Triton X-100
(f) 0.002 to 0.2% (w/v) CTAB.

The surfactant contained in the membrane permeabilization treatment agent of the present invention may be, for example, any of the following (a) to (c). More specifically, for example, the surfactant contained in the membrane permeabilization treatment agent of the present invention may be an aqueous solution containing any of the following (a) to (c):
(a) 0.05 to 0.5% (w/v) deoxycholate
(b) 0.01 to 1% (w/v) glycocholate
(c) 0.01 to 0.1% (w/v) SDS.

The organic solvent contained in the membrane permeabilization treatment agent of the present invention may be, for example, the following (a) or (b). More specifically, for example, the organic solvent may be an aqueous solution containing the following (a) or (b):
(a) 20 to 60% (v/v) ethanol
(b) 20 to 70% (v/v) acetone.

The membrane permeabilization treatment agent may be used, for example, for carrying out the method of the present invention, more specifically, for carrying out the membrane permeabilization step of the method of the present invention. Thus, the membrane permeabilization treatment agent of the present invention may be used, for example, as the membrane permeabilization treatment agent in the method of the present invention, more specifically, as the membrane permeabilization treatment agent in the membrane permeabilization step of the method of the present invention.

### <3> Detection Kit of Present Invention

The detection kit of the present invention is a detection kit for a particular protein contained in an extracellular vesicle.

The detection kit of the present invention may contain a carrier capable of binding to an extracellular-vesicle-specific marker present on the surface of an extracellular vesicle. To the carrier capable of binding to an extracellular-vesicle-specific marker present on the surface of an extracellular vesicle, contained in the detection kit of the present invention, descriptions on the carrier capable of binding to an extracellular-vesicle-specific marker present on the surface of an extracellular vesicle in the method of the present invention may be applied. The carrier capable of binding to an extracellular-vesicle-specific marker present on the surface of an extracellular vesicle, contained in the detection kit of the present invention may be a magnetic particle.

The detection kit of the present invention may contain the membrane permeabilization treatment agent of the present invention. The membrane permeabilization treatment agent of the present invention is as described above.

The detection kit of the present invention may contain a detection reagent. To the detection reagent contained in the detection kit of the present invention, descriptions on the detection reagent in the method of the present invention may be applied.

The detection kit of the present invention may be used, for example, for carrying out the method of the present invention.

### EXAMPLES

The present invention is described below more concretely using Examples and Comparative Examples. However, the present invention is not limited to these.

### Example 1

(1) Human embryonic kidney cells (293T cells) were cultured to confluence at 37°C under an environment of 5% CO₂ using D-MEM (Dulbecco's Modified Eagle's Medium) supplemented with 10% (v/v) FBS (fetal bovine serum) from which extracellular vesicles were removed. Thereafter, the culture supernatant (lot number 1) was collected, and then centrifuged at 10,000×g for 30 minutes. The resulting supernatant was centrifuged at 100,000xg for 70 minutes, and then the resulting pellet was suspended in PBS (Phosphate Buffered Saline), to obtain a concentrated suspension of extracellular vesicles released from the 293T cells.
(2) To 40 µL of a 10% (w/v) suspension of magnetic particles (manufactured by JSR Corporation) having a particle size of 2 to 3 µm, whose surface is modified with carboxy groups, an aqueous solution containing 0.5 mg of EDC (1-Ethyl-3-(3-Dimethylaminopropyl) Carbodiimide) was added, and the resulting mixture was stirred for 1 hour. After removing the reaction liquid, 20 µL of 1 mg/mL anti-CD9 antibody (manufactured by Frontier Institute Co., Ltd.) was added, and the resulting mixture was stirred for 3 hours. After removing the reaction liquid, the particles were immersed in 3% (w/v) aqueous BSA (Bovine serum albumin) solution for suppressing non-specific adsorption, to obtain an anti-CD9 antibody-bound magnetic particle suspension.
(3) In a well of a 96-well plate, 10 µL of the suspension containing 2% (w/v) anti-CD9 antibody-bound magnetic particles, obtained in (2), 5 µL of the concentrated suspension of extracellular vesicles, obtained in (1), and 90 µL of PBS supplemented with 3% (w/v) BSA were mixed, and the resulting mixture was stirred for 90 minutes. Thereafter, a magnet was placed close to the bottom of the 96-well plate to accumulate the magnetic particles in the bottom of the plate, and then the supernatant was removed, followed by three times of washing with PBS.
(4) After removing the washing liquid, 100 µL of an aqueous solution prepared by dissolving the anionic active agent sodium deoxycholate at 0.01% (w/v), 0.05% (w/v), 0.1% (w/v) 0.2% (w/v), or 0.5% (w/v) in PBS was added as a membrane permeabilization treatment agent, and the resulting mixture was stirred for 5 minutes. The supernatant was removed using a magnet in the same manner as in (3), and then the particles were washed three times with PBS.
(5) After removing the washing liquid, a mixture of 90 µL of PBS supplemented with 3% (w/v) BSA and 10 µL of FcR Blocking Reagent (manufactured by Miltenyi Biotec) was added as a blocking reagent, and the resulting mixture was stirred for 10 minutes.
(6) After removing the blocking reagent, 100 µL of PBS containing 2.5 µg/mL biotin-modified mouse-derived anti-human ALIX (Apoptosis-Linked gene 2-Interacting protein X; a protein present in extracellular vesicles released from 293T cells) antibody (manufactured by Santa Cruz) or 2.5 µg/mL biotin-modified mouse control antibody (mouse Isotype Control IgG, manufactured by Abcam), and containing 3% (w/v) BSA, was added, and the resulting mixture was stirred for 30 minutes. The supernatant was removed using a magnet in the same manner as in (3), and then the particles were washed three times with PBS.
(7) After removing the washing liquid, 100 µL of PBS containing streptavidin to which HRP (Horseradish Peroxidase) is polyvalently linked was added, and the resulting mixture was stirred for 20 minutes. The supernatant was removed using a magnet in the same manner as in (3), and then the particles were washed three times with PBS.
(8) After removing the washing liquid, 100 µL of a solution containing the chromogenic substrate TMB (tetramethylbenzidine) was added, and the resulting mixture was stirred for 20 minutes. Thereafter, 100 µL of an aqueous acidic solution containing 1 M hydrochloric acid was added to stop the enzyme reaction.
(9) After collecting 180 µL of the supernatant using a magnet, the supernatant was transferred to a new 96-well plate, and the absorbance at a wavelength of 450 nm was measured using a plate reader (manufactured by Tecan).

### Comparative Example 1

Color development of TMB was measured by the same method as in Example 1 except that 100 µL of PBS was added in Example 1 (4).

The results of Example 1 and Comparative Example 1 are shown together in Table 1. When PBS was added in the membrane permeabilization step (Comparative Example 1), membrane permeation of the extracellular vesicle did not occur, so that no difference in the absorbance was found between the anti-ALIX antibody and the mouse control antibody (anti-ALIX antibody: 0.04; mouse control antibody: 0.03). On the other hand, when PBS containing at least not less than 0.05% (w/v) sodium deoxycholate was added in the membrane permeabilization step (Example 1), a difference in the absorbance was found between the anti-ALIX antibody and the mouse control antibody. This indicates that, by the addition of sodium deoxycholate, at least part of the membrane of extracellular vesicle was solubilized to allow introduction of the anti-ALIX antibody into the extracellular vesicle and binding of the anti-ALIX antibody to ALIX present in the extracellular vesicle, resulting in the detection value (which has the same meaning as the absorbance value in the Examples in the present description). The mouse control antibody is not an IgG that specifically recognizes a human antigen. The systems in which sodium deoxycholate was added (Example 1) showed low absorbance values similarly to Comparative Example 1 (0.03 in both Example 1 and Comparative Example 1), indicating that the membrane permeabilization treatment with sodium deoxycholate did not cause binding of the non-specific mouse IgG It can therefore be said that the absorbance values obtained by the addition of the anti-ALIX antibody in Example 1 were not due to non-specific adsorption unique to the mouse IgG, but that the absorbance values reflect the amount of the anti-ALIX antibody that recognized ALIX present in the extracellular vesicle.

Although high absorbance values were found at the sodium deoxycholate concentrations within the range of 0.05% (w/v) to 0.2% (w/v), lower absorbance values were found outside this concentration range. It is assumed that, at the concentration lower than 0.05% (w/v), the membrane permeabilization step was insufficient, so that the antibody could not enter the inside, resulting in the low absorbance value, while at the concentration higher than 0.2% (w/v), the membrane permeabilization step itself was sufficient, but proteins including ALIX contained in the extracellular vesicle also leaked out of the vesicles, so that the amount of proteins remaining in the extracellular vesicle decreased, resulting in the low absorbance value.

**[Table 1]**

| | Sodium deoxycholate [% (w/v)] | Absorbance | |
|---|---|---|---|
| | | Anti-ALIX antibody | Mouse control antibody |
| Example 1 | 0.01 | 0.05 | 0.03 |
| | 0.05 | 0.40 | 0.03 |
| | 0.1 | 0.40 | 0.03 |
| | 0.2 | 0.36 | 0.03 |
| | 0.5 | 0.27 | 0.03 |
| Comparative Example 1 | 0 | 0.04 | 0.03 |

### Example 2

Color development of TMB was measured by the same method as in Example 1 except that an aqueous solution prepared by dissolving the nonionic surfactant Triton X-100 (trade name) at 0.001% (w/v), 0.005% (w/v), 0.01% (w/v), 0.05% (w/v), 0.1% (w/v), or 0.5% (w/v) in PBS was used as the membrane permeabilization treatment agent in Example 1(4).

The results of Example 2 are shown in Table 2. Since the absorbance value for the mouse control antibody did not depend on the Triton X-100 concentration, and was almost equivalent to the value in Comparative Example 1 (0.03 to 0.04) (Table 1), it can be seen that non-specific adsorption did not occur in the membrane permeabilization step. On the other hand, when at least not less than 0.01% (w/v) Triton X-100 was added in the membrane permeabilization step, the absorbance value for the anti-ALIX antibody was significantly higher than the value for the mouse control antibody, indicating that the membrane of the extracellular vesicle can be permeabilized also with a nonionic surfactant. Although high absorbance values were found at the Triton X-100 concentrations within the range of 0.01% (w/v) to 0.1% (w/v), lower absorbance values were found outside this range.

**[Table 2]**

| | Triton X-100 [% (w/v)] | Absorbance | |
|---|---|---|---|
| | | Anti-ALIX antibody | Mouse control antibody |
| Example 2 | 0.001 | 0.04 | 0.03 |
| | 0.005 | 0.07 | 0.03 |
| | 0.01 | 0.34 | 0.03 |
| | 0.05 | 0.38 | 0.04 |
| | 0.1 | 0.37 | 0.04 |
| | 0.5 | 0.24 | 0.04 |

### Example 3

Color development of TMB was measured by the same method as in Example 1 except that an aqueous solution prepared by dissolving the nonionic surfactant saponin at 0.01% (w/v), 0.1% (w/v), 0.25% (w/v), 1% (w/v), 2% (w/v), or 10% (w/v) in PBS was used as the membrane permeabilization treatment agent in Example 1 (4).

The results of Example 3 are shown in Table 3. Unlike the cases of sodium deoxycholate (Example 1) or Triton X-100 (Example 2), the absorbance value for the anti-ALIX antibody increased depending on the concentration of the saponin added. Further, the absorbance value for the mouse control antibody also increased depending on the concentration of saponin when the saponin concentration was not less than 2% (w/v), indicating occurrence of non-specific binding of the mouse IgG It can be judged that saponin can be used as the membrane permeabilization treatment agent in the present invention at least when the saponin concentration is within the range of 0.1% (w/v) to 1% (w/v) since, within this range, the absorbance value for the anti-ALIX antibody was significantly higher than the value for the mouse control antibody, and no increase in the absorbance value due to non-specific binding was found for the mouse control antibody.

**[Table 3]**

| | Saponin [% (w/v)] | Absorbance | |
|---|---|---|---|
| | | Anti-ALIX antibody | Mouse control antibody |
| Example 3 | 0.01 | 0.08 | 0.03 |
| | 0.1 | 0.19 | 0.03 |
| | 0.25 | 0.19 | 0.03 |
| | 1 | 0.19 | 0.04 |
| | 2 | 0.23 | 0.13 |
| | 10 | 0.30 | 0.24 |

### Example 4

Color development of TMB was measured by the same method as in Example 1 except that the concentrated suspension of extracellular vesicles released from 293T cells was obtained from a different lot (lot number 2) of culture supernatant in Example 1 (1), and that an aqueous solution prepared by dissolving the anionic surfactant sodium glycocholate at 0.01% (w/v), 0.05% (w/v), 0.08% (w/v), 0.1% (w/v), 0.2% (w/v), 0.3% (w/v), 0.4% (w/v), 0.5% (w/v), 0.6% (w/v), 0.8% (w/v), or 1% (w/v) in PBS was used as the membrane permeabilization treatment agent in Example 1 (4).

### Comparative Example 2

Color development of TMB was measured by the same method as in Example 1 except that the concentrated suspension of extracellular vesicles released from 293T cells was obtained from a different lot (lot number 2) of culture supernatant in Example 1 (1), and that 100 µL of PBS was added in Example 1 (4).

The results of Example 4 and Comparative Example 2 are shown together in Table 4. Since the absorbance value for the mouse control antibody did not depend on the sodium glycocholate concentration, and was equivalent to the value in Comparative Example 2 (0.02 in both cases), it can be seen that non-specific adsorption due to the membrane permeabilization step did not occur. On the other hand, when at least not less than 0.05% (w/v) sodium glycocholate was added in the membrane permeabilization step, the absorbance value for the anti-ALIX antibody was significantly higher than the value for the mouse control antibody, indicating that the membrane of the extracellular vesicle can be permeabilized also with sodium glycocholate.

The concentrations of sodium glycocholate at which high absorbance values were found were 0.05% (w/v) to 0.8% (w/v), which were higher than the concentrations in the cases of sodium deoxycholate (0.05% (w/v) to 0.2% (w/v)), which is similarly an anionic surfactant. This is assumed to be due to a higher critical micelle concentration of sodium glycocholate relative to that of sodium deoxycholate (sodium deoxycholate: 5 mM; sodium glycocholate: 13 mM).

**[Table 4]**

| | Sodium glycocholate [% (w/v)] | Absorbance | |
|---|---|---|---|
| | | Anti-ALIX antibody | Mouse control antibody |
| Example 4 | 0.01 | 0.06 | 0.02 |
| | 0.05 | 0.23 | 0.02 |
| | 0.08 | 0.28 | 0.02 |
| | 0.1 | 0.32 | 0.02 |
| | 0.2 | 0.36 | 0.02 |
| | 0.3 | 0.39 | 0.02 |
| | 0.4 | 0.45 | 0.02 |
| | 0.5 | 0.55 | 0.02 |
| | 0.6 | 0.50 | 0.02 |
| | 0.8 | 0.45 | 0.02 |
| | 1 | 0.14 | 0.02 |
| Comparative Example 2 | 0 | 0.04 | 0.02 |

### Example 5

Color development of TMB was measured by the same method as in Example 1 except that the concentrated suspension of extracellular vesicles released from 293T cells was obtained from a different lot (lot number 2) of culture supernatant in Example 1 (1), and that an aqueous solution prepared by dissolving the anionic surfactant SDS (sodium dodecyl sulfate) at 0.01% (w/v), 0.02% (w/v), 0.1% (w/v), 0.2% (w/v), 0.5% (w/v), or 1% (w/v) in PBS was used as the membrane permeabilization treatment agent in Example 1 (4).

The results of Example 5 are shown in Table 5. Since the absorbance value for the mouse control antibody did not depend on SDS, and was equivalent to the value in Comparative Example 2 (0.02 in both cases) (Table 4), it can be seen that non-specific adsorption due to the membrane permeabilization step did not occur. On the other hand, when 0.02% (w/v) SDS was added in the membrane permeabilization step, the absorbance value for the anti-ALIX antibody was significantly higher than the value for the mouse control antibody, indicating that the membrane of the extracellular vesicle can be permeabilized also with SDS.

The only concentration of SDS at which a high absorbance value was found was 0.02% (w/v), indicating a narrower optimum concentration range than those of sodium deoxycholate (0.05% (w/v) to 0.2% (w/v)) and sodium glycocholate (0.05% (w/v) to 0.8% (w/v)), which are similarly anionic surfactants. This is assumed to be due to a higher protein-denaturing capacity of SDS relative to those of sodium deoxycholate and sodium glycocholate.

**[Table 5]**

| | SDS [% (w/v)] | Absorbance | |
|---|---|---|---|
| | | Anti-ALIX antibody | Mouse control antibody |
| Example 5 | 0.01 | 0.04 | 0.02 |
| | 0.02 | 0.23 | 0.02 |
| | 0.1 | 0.05 | 0.02 |
| | 0.2 | 0.05 | 0.02 |
| | 0.5 | 0.05 | 0.02 |
| | 1 | 0.06 | 0.02 |

### Example 6

Color development of TMB was measured by the same method as in Example 1 except that the concentrated suspension of extracellular vesicles released from 293T cells was obtained from a different lot (lot number 3) of culture supernatant in Example 1 (1), and that an aqueous solution prepared by dissolving the cationic surfactant CTAB (cetyltrimethylammonium bromide) at 0.001% (w/v), 0.002% (w/v), 0.01% (w/v), 0.05% (w/v), or 0.2% (w/v) in PBS was used as the membrane permeabilization treatment agent in Example 1 (4).

### Comparative Example 3

Color development of TMB was measured by the same method as in Example 1 except that the concentrated suspension of extracellular vesicles released from 293T cells was obtained from a different lot (lot number 3) of culture supernatant in Example 1 (1), and that 100 µL of PBS was added in Example 1 (4).

The results of Example 6 and Comparative Example 3 are shown together in Table 6. Since the absorbance value for the mouse control antibody did not depend on CTAB, and was equivalent to the value in Comparative Example 3 (0.02 in both cases), it can be seen that non-specific adsorption due to the membrane permeabilization step did not occur. On the other hand, when CTAB was added at at least 0.002% (w/v) in the membrane permeabilization step, the absorbance value for the anti-ALIX antibody was significantly higher than the value for the mouse control antibody, indicating that the membrane of the extracellular vesicle can be permeabilized also with a cationic surfactant. Although high absorbance values were found at the CTAB concentrations within the range of 0.002% (w/v) to 0.05% (w/v), lower absorbance values were found outside this range.

**[Table 6]**

| | CTAB [% (w/v)] | Absorbance | |
|---|---|---|---|
| | | Anti-ALIX antibody | Mouse control antibody |
| Example 6 | 0.001 | 0.03 | 0.02 |
| | 0.002 | 0.17 | 0.02 |
| | 0.01 | 0.18 | 0.02 |
| | 0.05 | 0.15 | 0.02 |
| | 0.2 | 0.11 | 0.02 |
| Comparative Example 3 | 0 | 0.03 | 0.02 |

### Example 7

Color development of TMB was measured by the same method as in Example 1 except that the concentrated suspension of extracellular vesicles released from 293T cells was obtained from a different lot (lot number 4) of culture supernatant in Example 1 (1), and that an aqueous solution prepared by dissolving one of the following (a) to (g) in PBS was used as the membrane permeabilization treatment agent in Example 1 (4). The concentrations of (a) to (f) added are the optimum concentrations of the surfactants, obtained by the results of Examples 1 to 6.
(a) 0.02% (w/v) SDS
(b) 0.5% (w/v) Sodium glycocholate
(c) 0.075% (w/v) Sodium deoxycholate
(d) 0.01% (w/v) CTAB
(e) 0.25% (w/v) Saponin
(f) 0.02% (w/v) Triton X-100
(g) 95% (v/v) Ethanol

### Comparative Example 4

Color development of TMB was measured by the same method as in Example 1 except that the concentrated suspension of extracellular vesicles released from 293T cells was obtained from a different lot (lot number 4) of culture supernatant in Example 1 (1), and that 100 µL of PBS was added in Example 1 (4).

### Comparative Example 5

Color development of TMB was measured by the same method as in Example 1 except that the concentrated suspension of extracellular vesicles released from 293T cells was obtained from a different lot (lot number 4) of culture supernatant in Example 1 (1), and that sonication treatment was carried out for 5 minutes after addition of 100 µL of PBS in Example 1 (4).

The results of Example 7, and Comparative Examples 4 and 5 are shown together in Table 7. As a result of using the concentrated suspensions of extracellular vesicles of the same lot, and carrying out the membrane permeabilization step with the surfactants at their optimum concentrations, the absorbance value was found to be higher in the cases where an anionic surfactant was used ((a) SDS: 1.10; (b) sodium glycocholate: 1.12; (c) sodium deoxycholate: 1.13) than in the cases where a nonionic surfactant ((e) saponin: 0.77; (f) Triton X-100: 0.78) or a cationic surfactant ((d) CTAB: 0.82) was used. These results indicate that, among the surfactants, anionic surfactants are more suitable than other surfactants as the membrane permeabilization treatment agent used in the present invention, from the viewpoint of membrane permeability of the extracellular vesicle and suppression of leakage of proteins contained in the membrane-permeabilized extracellular vesicle.

It can be seen that use of the organic solvent ethanol (g) also enables permeabilization of the membrane of extracellular vesicle since the absorbance value for the anti-ALIX antibody (0.28) was higher than the value for the mouse control antibody (0.04) and also higher than the absorbance value for the anti-ALIX antibody in Comparative Example 4 (0.10). However, the absorbance value for the anti-ALIX antibody was lower than the values in the cases where the surfactants were added (0.77 to 1.13). This is assumed to be due to the fact that the magnetic particles aggregated upon the addition of ethanol, resulting in a decrease in the reaction efficiency after the membrane permeabilization step.

In the case where sonication treatment was carried out instead of the membrane permeabilization step (Comparative Example 5), the absorbance value for the anti-ALIX antibody was 0.25. This value was higher than the value in Comparative Example 4 (0.10), but lower than the values in the cases where the surfactants were added (0.77 to 1.13). This is assumed to be due to the fact that membrane disruption of the extracellular vesicle by the treatment was insufficient, and that the bond between the extracellular vesicle and the magnetic particle was broken by the treatment.

**[Table 7]**

| | | Membrane permeabilization treatment agent | Absorbance | |
|---|---|---|---|---|
| | | | Anti-ALIX antibody | Mouse control antibody |
| Example 7 | (a) | 0.02% (w/v) SDS | 1.10 | - |
| | (b) | 0.5% (w/v) Sodium glycocholate | 1.12 | - |
| | (c) | Sodium | 1.13 | - |
| | (d) | 0.01% (w/v) CTAB | 0.82 | - |
| | (e) | 0.25% (w/v) Saponin | 0.77 | - |
| | (f) | 0.02% (w/v) Triton X-100 | 0.78 | - |
| | (g) | 95% (v/v) Ethanol | 0.28 | 0.05 |
| Comparative Example 4 | | PBS | 0.10 | 0.04 |
| Comparative Example 5 | | Sonication | 0.25 | 0.04 |

### Example 8

Color development of TMB was measured by the same method as in Example 1 except that the concentrated suspension of extracellular vesicles released from 293T cells was obtained from a different lot (lot number 4) of culture supernatant in Example 1 (1), and that Example 1 (4) was carried out by removing the washing liquid, adding 100 µL of PBS supplemented with 1% (w/v) formaldehyde as a fixative, stirring the resulting mixture for 5 minutes, removing the supernatant using a magnet, adding 100 µL of PBS supplemented with 0.075% (w/v) sodium deoxycholate as a membrane permeabilization treatment agent, and then stirring again the mixture for 5 minutes.

### Example 9

Color development of TMB was measured by the same method as in Example 1 except that the concentrated suspension of extracellular vesicles released from 293T cells was obtained from a different lot (lot number 4) of culture supernatant in Example 1 (1), and that 100 µL of an aqueous PBS solution prepared by dissolving formaldehyde as a fixative and sodium deoxycholate as a membrane permeabilization treatment agent at one of the concentrations (a) to (f) in PBS was added in Example 1 (4).
(a) 1% (w/v) Formaldehyde + 0.05% (w/v) Sodium deoxycholate
(b) 1% (w/v) Formaldehyde + 0.075% (w/v) Sodium deoxycholate
(c) 1% (w/v) Formaldehyde + 0.1% (w/v) Sodium deoxycholate
(d) 1% (w/v) Formaldehyde + 0.2% (w/v) Sodium deoxycholate
(e) 1% (w/v) Formaldehyde + 0.5% (w/v) Sodium deoxycholate
(f) 4% (w/v) Formaldehyde + 0.1% (w/v) Sodium deoxycholate

### Comparative Example 6

Color development of TMB was measured by the same method as in Example 1 except that the concentrated suspension of extracellular vesicles released from 293T cells was obtained from a different lot (lot number 4) of culture supernatant in Example 1 (1), and that 100 µL of PBS supplemented with 1% (w/v) or 4% (w/v) formaldehyde was added in Example 1 (4).

The results of Examples 8 and 9, and Comparative Examples 4 and 6 are shown together in Table 8. Even in the cases where the step of adding formaldehyde as a fixative (fixation step) was included, the absorbance value for the anti-ALIX antibody (0.47 to 1.05) was higher than the value for the mouse control antibody (0.04) in all cases. It can thus be seen that the membrane of the extracellular vesicle could be permeabilized (Examples 8 and 9). It can also be seen that, by carrying out the fixation step and the step of adding sodium deoxycholate as a membrane permeabilization treatment agent (membrane permeabilization step) at the same time (Example 9 (b)), the absorbance value for the anti-ALIX antibody was improved compared to the case where the membrane permeabilization step was carried out after the fixation step (Example 8).

On the other hand, when only the fixation step was carried out (Comparative Example 6), the absorbance value for the anti-ALIX antibody was almost equivalent to the value in the case where only PBS was added (that is, in the case where neither the fixation step nor the membrane permeabilization step was carried out; Comparative Example 4) (Comparative Example 4: 0.10; Comparative Example 6: 0.10 to 0.11).

In Example 9, the decrease in the absorbance value that occurred in Example 1 (Table 1) did not occur even by increasing the sodium deoxycholate concentration to 0.5% (w/v). This is assumed to be due to the fact that leakage of proteins contained in the extracellular vesicle could be suppressed by the addition of the fixative formaldehyde. Further, when the formaldehyde concentration was set to 4% (w/v) without changing the sodium deoxycholate concentration, a higher detection value was found compared to the case where the formaldehyde concentration was 1% (w/v) (Example 9 (c): 0.85; Example 9 (f): 1.05). This result also demonstrates the effect of the fixative formaldehyde to suppress the protein leakage.

**[Table 8]**

| | | Formaldehyde concentration [% (w/v)] | Sodium deoxycholate concentration [% (w/v)] | Fixation-membrane permeabilization treatment method | Absorbance | |
|---|---|---|---|---|---|---|
| | | | | | Anti-ALIX antibody | Mouse control antibody |
| Example 8 | | 1 | 0.075 | Fixation followed by membrane permeabilization | 0.47 | 0.04 |
| Example 9 | (a) | 1 | 0.05 | Simultaneous treatment | 0.49 | - |
| | (b) | 1 | 0.075 | Simultaneous treatment | 0.92 | 0.04 |
| | (c) | 1 | 0.1 | Simultaneous treatment | 0.85 | - |
| | (d) | 1 | 0.2 | Simultaneous treatment | 0.86 | - |
| | (e) | 1 | 0.5 | Simultaneous treatment | 0.98 | 0.04 |
| | (f) | 4 | 0.1 | Simultaneous treatment | 1.05 | 0.05 |
| Comparative Example 4 | | - | - | Simultaneous treatment | 0.10 | 0.04 |
| Comparative Example 6 | | 1 | - | Simultaneous treatment | 0.11 | 0.04 |
| | | 4 | - | Simultaneous treatment | 0.10 | 0.04 |

### Example 10

Color development of TMB was measured by the same method as in Example 1 except that the concentrated suspension of extracellular vesicles released from 293T cells was obtained from a different lot (lot number 4) of culture supernatant in Example 1 (1), and that 100 µL of an aqueous solution prepared by dissolving ethanol as a fixative and as a membrane permeabilization treatment agent and sodium deoxycholate as a membrane permeabilization treatment agent at one of the concentrations (a) to (h) in PBS was added in Example 1 (4).
(a) 5% (v/v) Ethanol + 0.1% (w/v) Sodium deoxycholate
(b) 10% (v/v) Ethanol + 0.1% (w/v) Sodium deoxycholate
(c) 25% (v/v) Ethanol + 0.1% (w/v) Sodium deoxycholate
(d) 50% (v/v) Ethanol + 0.1% (w/v) Sodium deoxycholate
(e) 5% (v/v) Ethanol
(f) 10% (v/v) Ethanol
(g) 25% (v/v) Ethanol
(h) 50% (v/v) Ethanol

The results of Example 10 are shown in Table 9. When 25% (v/v) ethanol or 50% (v/v) ethanol was used in combination with 0.1% (w/v) sodium deoxycholate (Example 10 (c) and (d)), the absorbance value was higher than the value in the case where sodium deoxycholate was used alone (Example 7 (c), Table 7) (Example 7 (c): 1.13; Example 10 (c): 1.58; Example 10 (d): 1.40). On the other hand, when 25% (v/v) ethanol was used alone (Example 10 (g)), the absorbance value was 0.16, indicating insufficient membrane permeabilization effect. It is thus assumed that, by the simultaneous occurrence of insolubilization of protein by ethanol (fixation step) and membrane permeabilization by sodium deoxycholate (membrane permeabilization step), leakage of proteins from the inside of the extracellular vesicle was largely suppressed, resulting in high detection values (absorbance values).

**[Table 9]**

| | | Ethanol concentration [% (v/v)] | Sodium deoxycholate concentration [% (w/v)] | Fixation-membrane permeabilization treatment method | Absorbance | |
|---|---|---|---|---|---|---|
| | | | | | Anti-ALIX antibody | Mouse control antibody |
| Example 10 | (a) | 5 | 0.1 | Simultaneous treatment | 0.95 | 0.04 |
| | (b) | 10 | 0.1 | Simultaneous treatment | 1.00 | 0.04 |
| | (c) | 25 | 0.1 | Simultaneous treatment | 1.58 | 0.04 |
| | (d) | 50 | 0.1 | Simultaneous treatment | 1.40 | 0.06 |
| | (e) | 5 | - | Simultaneous treatment | 0.06 | 0.04 |
| | (f) | 10 | - | Simultaneous treatment | 0.06 | 0.04 |
| | (g) | 25 | - | Simultaneous treatment | 0.16 | 0.04 |
| | (h) | 50 | - | Simultaneous treatment | 0.97 | 0.07 |

### Example 11

Color development of TMB was measured by the same method as in Example 1 except that the concentrated suspension of extracellular vesicles released from 293T cells was obtained from a different lot (lot number 5) of culture supernatant in Example 1 (1), and that 100 µL of an aqueous solution prepared by dissolving ethanol alone as a fixative and as a membrane permeabilization treatment agent at 40% (v/v), or dissolving ethanol at 40% (v/v) and sodium deoxycholate as a membrane permeabilization treatment agent at 0.0001% (w/v), 0.001% (w/v), 0.01% (w/v), 0.02% (w/v), 0.05% (w/v), 0.1% (w/v), 0.2% (w/v), or 0.5% (w/v) in PBS was added in Example 1 (4).

The results of Example 11 are shown in Table 10. It can be seen, in the present Example, that the membrane permeabilization of the extracellular vesicle was appropriately carried out since the absorbance value for the anti-ALIX antibody (0.46 to 0.72) was higher than the value for the mouse control antibody (0.04) in all cases. Further, when 0.001% (w/v) to 0.2% (w/v) sodium deoxycholate was added to 40% (v/v) ethanol, the absorbance value for the anti-ALIX antibody was improved (0.64 to 0.72) compared to the case where the fixation step and the membrane permeabilization step were carried out with 40% (v/v) ethanol alone (0.46). It is assumed that, when the concentration of sodium deoxycholate was higher than 0.2% (w/v), insufficient insolubilization of proteins by ethanol led to leakage of the proteins from the inside of the extracellular vesicle.

As shown in Example 1 (Table 1), when the membrane permeabilization step was carried out with sodium deoxycholate alone, membrane permeabilization of the extracellular vesicle was hardly achieved at not more than 0.01% (w/v) (absorbance value, 0.05). On the other hand, in the present Example, wherein 40% (v/v) ethanol was also added, the absorbance value for the anti-ALIX antibody could be improved even with not more than 0.01% (w/v) sodium deoxycholate (0.70 at either 0.001% (w/v) or 0.01% (w/v)) compared to the case where the fixation step and the membrane permeabilization step were carried out with 40% (v/v) ethanol alone (0.46). It can thus be seen that, even within a surfactant concentration range in which the membrane permeabilization effect on extracellular vesicle can be hardly obtained, improvement of the detection value (absorbance value) may be possible by addition of ethanol as a fixative and as a membrane permeabilization treatment agent.

**[Table 10]**

| | Ethanol concentration [% (v/v)] | Sodium deoxycholate concentration [% (w/v)] | Fixation-membrane permeabilization treatment method | Absorbance | |
|---|---|---|---|---|---|
| | | | | Anti-ALIX antibody | Mouse control antibody |
| Example 11 | 40 | 0 | Simultaneous treatment | 0.46 | 0.04 |
| | 40 | 0.0001 | Simultaneous treatment | 0.50 | - |
| | 40 | 0.001 | Simultaneous treatment | 0.70 | 0.04 |
| | 40 | 0.01 | Simultaneous treatment | 0.70 | 0.04 |
| | 40 | 0.02 | Simultaneous treatment | 0.70 | 0.04 |
| | 40 | 0.05 | Simultaneous treatment | 0.72 | 0.04 |
| | 40 | 0.1 | Simultaneous treatment | 0.71 | 0.04 |
| | 40 | 0.2 | Simultaneous treatment | 0.64 | 0.04 |
| | 40 | 0.5 | Simultaneous treatment | 0.48 | 0.04 |

### Example 12

Color development of TMB was measured by the same method as in Example 1 except that the concentrated suspension of extracellular vesicles released from 293T cells was obtained from a different lot (lot number 6) of culture supernatant in Example 1 (1), and that 100 µL of an aqueous solution prepared by dissolving ethanol at 40% (w/v) as a fixative and as a membrane permeabilization treatment agent and one of the surfactants (a) to (c) as a membrane permeabilization treatment agent in PBS was added in Example 1 (4).
(a) 0.1% (w/v) Sodium deoxycholate
(b) 0.05% (w/v) Triton X-100
(c) 0.01% (w/v) CTAB

The results of Example 12 are shown in Table 11. When the anionic surfactant sodium deoxycholate was used as the surfactant to be mixed with 40% (w/v) ethanol, the absorbance value for the anti-ALIX antibody was higher (sodium deoxycholate: 0.82) than the value in the cases where Triton X-100 (nonionic surfactant) or CTAB (cationic surfactant) was used (Triton X-100: 0.63; CTAB: 0.38).

**[Table 11]**

| | Fixation-membrane permeabilization treatment agent | Fixation-membrane permeabilization treatment method | Absorbance | |
|---|---|---|---|---|
| | | | Anti-ALIX antibody | Mouse control antibody |
| Example 12 | 40% (v/v) Ethanol + 0.1% (w/v) Sodium deoxycholate | Simultaneous treatment | 0.82 | 0.04 |
| | 40% (v/v) Ethanol + 0.05% (w/v) Triton X-100 | Simultaneous treatment | 0.63 | 0.04 |
| | 40% (v/v) Ethanol + 0.01% (w/v) CTAB | Simultaneous treatment | 0.38 | 0.04 |

### Example 13

Color development of TMB was measured by the same method as in Example 1 except that the concentrated suspension of extracellular vesicles released from 293T cells was obtained from a different lot (lot number 7) of culture supernatant in Example 1 (1), that 0.5 µL of the concentrated suspension of extracellular vesicles was used in Example 1 (3), that 100 µL of an aqueous solution prepared by dissolving ethanol as a fixative and as a membrane permeabilization treatment agent at one of the concentrations (a) to (g) in PBS was added in Example 1 (4), and that a plate reader manufactured by Corona Electric Co., Ltd. was used in Example 1 (9).
(a) 20% (v/v) Ethanol
(b) 30% (v/v) Ethanol
(c) 40% (v/v) Ethanol
(d) 50% (v/v) Ethanol
(e) 60% (v/v) Ethanol
(f) 70% (v/v) Ethanol
(g) 95% (v/v) Ethanol

### Comparative Example 7

Color development of TMB was measured by the same method as in Example 13 except that PBS was used as the fixative and as the membrane permeabilization treatment agent in Example 13.

The results of Example 13 and Comparative Example 7 are shown together in Table 12. The relative value of the absorbance for the anti-ALIX antibody with respect to the absorbance for the mouse control antibody was higher in the cases where the organic solvent ethanol was used (Example 13: 1.472 to 3.408), compared to the case where ethanol was not added (Comparative Example 7: 1.470). The relative value was especially high (3.363 to 3.408) when the ethanol concentration was within the range of 30% to 50%. The presence of the optimum concentration range is assumed to be due to the fact that the low ethanol concentration led to insufficient membrane permeability of the extracellular vesicle, while that the high ethanol concentration led to poor dispersion stability of the magnetic particles.

**[Table 12]**

| | | Ethanol concentration [% (v/v)] | Absorbance | | Relative value |
|---|---|---|---|---|---|
| | | | Anti-ALIX antibody | Mouse control antibody | |
| Example 13 | (a) | 20 | 0.405 | 0.194 | 2.088 |
| | (b) | 30 | 0.629 | 0.187 | 3.364 |
| | (c) | 40 | 0.609 | 0.180 | 3.383 |
| | (d) | 50 | 1.145 | 0.336 | 3.408 |
| | (e) | 60 | 0.683 | 0.357 | 1.913 |
| | (f) | 70 | 0.437 | 0.284 | 1.539 |
| | (g) | 95 | 0.315 | 0.214 | 1.472 |
| Comparative Example 7 | | 0 | 0.294 | 0.200 | 1.470 |

| | | | | | |
|---|---|---|---|---|---|
| Relative value: absorbance for anti-ALIX antibody / absorbance for mouse control antibody | | | | | |

### Example 14

Color development of TMB was measured by the same method as in Example 1 except that the concentrated suspension of extracellular vesicles released from 293T cells was obtained from a different lot (lot number 7) of culture supernatant in Example 1 (1), that 0.5 µL of the concentrated suspension of extracellular vesicles was used in Example 1 (3), that 100 µL of an aqueous solution prepared by dissolving acetone as a fixative and as a membrane permeabilization treatment agent at one of the concentrations (a) to (f) in PBS was added in Example 1 (4), and that a plate reader manufactured by Corona Electric Co., Ltd. was used in Example 1 (9).
(a) 20% (v/v) Acetone
(b) 30% (v/v) Acetone
(c) 40% (v/v) Acetone
(d) 60% (v/v) Acetone
(e) 70% (v/v) Acetone
(f) 99% (v/v) Acetone

The results of Example 14 are shown in Table 13. The relative value of the absorbance for the anti-ALIX antibody with respect to the absorbance for the mouse control antibody was higher in the cases where 20 to 70% acetone, which is an organic solvent, was used (Example 14: 2.75 to 3.75), compared to the case where acetone was not added (Comparative Example 7: 1.47). When 99% acetone was used, the evaluation was impossible since the 96-well plate melted. The relative value was especially high (3.30 to 3.75) when the acetone concentration was within the range of 30% to 70%. It is assumed that the low acetone concentration led to insufficient membrane permeability of the extracellular vesicle, resulting in the low value.

**[Table 13]**

| | | Acetone concentration [% (v/v)] | Absorbance | | Relative value |
|---|---|---|---|---|---|
| | | | Anti-ALIX antibody | Mouse control antibody | |
| Example 14 | (a) | 20 | 0.58 | 0.21 | 2.75 |
| | (b) | 30 | 0.73 | 0.22 | 3.30 |
| | (c) | 40 | 1.25 | 0.34 | 3.74 |
| | (d) | 60 | 0.99 | 0.26 | 3.75 |
| | (e) | 70 | 0.89 | 0.25 | 3.63 |
| | (f) | 99 | Unmeasurable | Unmeasurable | - |

| | | | | | |
|---|---|---|---|---|---|
| Relative value: absorbance for anti-ALIX antibody / absorbance for mouse control antibody | | | | | |

### Example 15

Color development of TMB was measured by the same method as in Example 1 except that the concentrated suspension of extracellular vesicles released from 293T cells was obtained from a different lot (lot number 8) of culture supernatant in Example 1 (1), that 0.5 µL of the concentrated suspension of extracellular vesicles was used in Example 1 (3), that 100 µL of an aqueous solution containing the surfactant or organic solvent of one of (a) to (e) as a fixative and/or membrane permeabilization treatment agent was added in Example 1 (4), and that a plate reader manufactured by Corona Electric Co., Ltd. was used in Example 1 (9). Each of the concentrations of (a) to (e) added is the optimum concentration of the surfactant or organic solvent, obtained by the results of Examples 1 to 6, 13, and 14.
(a) 0.075% (w/v) Sodium deoxycholate
(b) 0.02% (w/v) Triton X-100
(c) 0.01% (w/v) CTAB
(d) 40% (v/v) Ethanol
(e) 40% (v/v) Acetone

The results of Example 15 are shown in Table 14. The relative value of the absorbance for the anti-ALIX antibody with respect to the absorbance for the mouse control antibody was higher in the cases where an organic solvent was used (ethanol: 7.56; acetone: 4.10), compared to the cases where a surfactant was used (sodium deoxycholate: 2.72; Triton X-100: 2.59; CTAB:1.49). The surfactant with the highest relative value was the anionic surfactant sodium deoxycholate, and the organic solvent with the highest relative value was ethanol.

**[Table 14]**

| | | Membrane permeabilization treatment agent | Absorbance | | Relative value |
|---|---|---|---|---|---|
| | | | Anti-ALIX antibody | Mouse control antibody | |
| Example 15 | (a) | 0.075% (w/v) Sodium deoxycholate | 0.29 | 0.11 | 2.72 |
| | (b) | 0.02% (w/v) Triton X-100 | 0.44 | 0.17 | 2.59 |
| | (c) | 0.01% (w/v) CTAB | 0.32 | 0.21 | 1.49 |
| | (d) | 40% (v/v) Ethanol | 1.18 | 0.16 | 7.56 |
| | (e) | 40% (v/v) Acetone | 1.03 | 0.25 | 4.10 |

| | | | | | |
|---|---|---|---|---|---|
| Relative value: absorbance for anti-ALIX antibody / absorbance for mouse control antibody | | | | | |

### Example 16

Color development of TMB was measured by the same method as in Example 15 except that the volume of the extracellular vesicle suspension used in Example 15 was 2 µL, and that 2.5 µg/mL biotin-modified mouse-derived anti-human HSP70 (Heat shock protein 70; a protein present in extracellular vesicle released from 293T cells) antibody (manufactured by StressMarq Biosciences Inc.) or 2.5 µg/mL biotin-modified mouse control antibody (mouse Isotype Control IgG, manufactured by Abcam) was used as the antibody in Example 15.

The results of Example 16 are shown in Table 15. The relative value of the absorbance for the anti-ALIX antibody with respect to the absorbance for the mouse control antibody hardly changed even by the use of an organic solvent (ethanol: 2.37; acetone: 1.95), compared to the cases where a surfactant was used (sodium deoxycholate: 2.46; Triton X-100: 1.40; CTAB:1.66). The surfactant with the highest relative value was the anionic surfactant sodium deoxycholate, and the organic solvent with the highest relative value was ethanol. From the present measurement results for HSP70 and the measurement results for ALIX (Example 15), it can be seen that highly sensitive measurement is possible when an anionic surfactant is used as a surfactant, or when ethanol is used as an organic solvent, irrespective of the type of the particular protein to be measured.

**[Table 15]**

| | | Membrane permeabilization treatment agent | Absorbance | | Relative value |
|---|---|---|---|---|---|
| | | | Anti-HSP70 antibody | Mouse control antibody | |
| Example 16 | (a) | 0.075% (w/v) Sodium deoxycholate | 0.30 | 0.12 | 2.46 |
| | (b) | 0.02% (w/v) Triton X-100 | 0.19 | 0.13 | 1.40 |
| | (c) | 0.01% (w/v) CTAB | 0.25 | 0.15 | 1.66 |
| | (d) | 40% (v/v) Ethanol | 0.36 | 0.15 | 2.37 |
| | (e) | 40% (v/v) Acetone | 0.40 | 0.21 | 1.95 |

| | | | | | |
|---|---|---|---|---|---|
| Relative value: absorbance for anti-HSP70 antibody / absorbance for mouse control antibody | | | | | |

### Comparative Example 8

(1) To 0.5 µL (5 µL after addition of 4.5 µL of PBS) or 5 µL of the same extracellular vesicle suspension as in Example 13 (lot number 7), 1.25 µL of a loading buffer (250 mM Tris-HCl (pH 6.8), 8% SDS, 0.1% bromophenol blue, 40% Glycerol, 100 mM DTT) was added, and the resulting mixture was heated at 95°C for 5 minutes to cause membrane permeabilization of the extracellular vesicle, and to solubilize and denature proteins in the vesicles.
(2) The sample prepared in (1) was subjected to electrophoresis (30 mA, 40 minutes) using a polyacrylamide gel (catalog number: 4561096; manufactured by Bio-Rad Laboratories, Inc.), to separate proteins.
(3) The proteins separated by the gel in (2) was transblotted to a PVDF membrane (catalog number: 1704156; manufactured by Bio-Rad Laboratories, Inc.), and blotting was carried out using a Trans-Blot Turbo Transfer System (manufactured by Bio-Rad Laboratories, Inc.).
(4) The membrane blotted in (3) was subjected to blocking treatment using Blocking One (manufactured by Nacalai Tesque, Inc.), and then immersed in a solution containing Blocking One diluted 20-fold with TBST (Tris Buffered Saline supplemented with 0.1% Tween 20) and 1 µg/mL anti-ALIX antibody at room temperature for 60 minutes.
(5) The membrane in (4) was washed with TBST, and then immersed in a solution containing Blocking One diluted 20-fold with TBST and an HRP-modified goat anti-mouse IgG antibody (catalog number: 31430; manufactured by ThermoFisher) diluted 10,000-fold, at room temperature for 60 minutes.
(6) The membrane of (5) was washed with TBST, and then subjected to color development using SuperSignal West Pico PLUS Chemiluminescent Substrate (catalog number: 34580; manufactured by ThermoFisher), followed by taking a picture of the membrane to confirm the ALIX band.

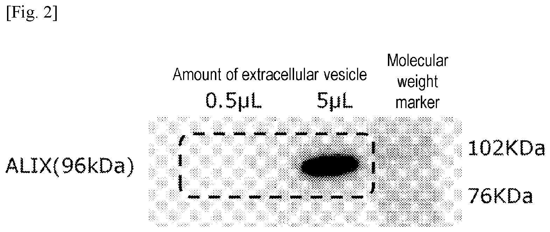

The results of Comparative Example 8 are shown in Fig. 2. When the volume of the extracellular vesicle suspension was 0.5 µL, especially in the cases where the ethanol concentration was 30% to 50% in Example 13 (the relative value of the absorbance for the anti-ALIX antibody with respect to the absorbance for the mouse control antibody: 3.363 to 3.408), ALIX in the extracellular vesicle could be sensitively detected. On the other hand, based on the results of Western blotting in Comparative Example 16, although the band derived from ALIX could be detected when the volume of the extracellular vesicle suspension was 5 µL, the band could not be detected when the volume was 0.5 µL.

Even when the amount of the extracellular vesicle was too small for the detection by the method of Patent Document 3, in which detection by Western blotting is carried out after allowing leakage of proteins from extracellular vesicle, the present detection enabled highly sensitive detection of the protein in the extracellular vesicle by detecting the protein without allowing its leakage.

### Example 17

(1) A concentrated suspension of extracellular vesicles released from 293T cells was obtained by the same method as in Example 1 (1) except that a different lot (lot number 9) of culture supernatant was used.
(2) Membrane permeabilization treatment of the extracellular vesicle was carried out by the same method as in Example 1 (2) to (4) except that 0.5 µL of the concentrated suspension of extracellular vesicles obtained in (1) was used, and that 100 µL of an aqueous solution containing 40% (v/v) ethanol was used as the membrane permeabilization treatment agent.
(3) HRP-modified streptavidin was bound by the same method as in Example 1 (5) to (7) except that, after removing the washing liquid, 100 µL of an activation reagent, (a) to (c), was added followed by stirring the resulting mixture for 10 minutes, removing the supernatant using a magnet in the same manner as in Example 1 (3), performing three times of washing with PBS, and then performing blocking treatment.
   (a) PBS (control for the activation reagent)
   (b) Proteinase K diluted 50-fold with PBS (catalog number: 9034; manufactured by Takara Bio Inc.)
   (c) HistoReveal (manufactured by Abcam)
(4) After removing the washing liquid, 150 µL of the substrate reaction liquid of SuperSignal ELISA Pico Chemiluminescent Substrate (manufactured by ThermoFisher) was added as a chromogenic substrate, and then the amount of luminescence was measured using a plate reader (manufactured by Tecan) within 5 minutes.

### Example 18

The amount of luminescence was measured by the same method as in Example 17 except that, in Example 17 (2), the treatment with the fixative and the membrane permeabilization treatment agent was carried out by adding 100 µL of PBS supplemented with 1% or 4% (w/v) formaldehyde, stirring the resulting mixture for 5 minutes, removing the supernatant using a magnet, adding 100 µL of an aqueous solution containing 40% (v/v) ethanol as a fixative and as a membrane permeabilization treatment agent, and then stirring again the mixture for 5 minutes, and that 100 µL of PBS or Proteinase K diluted 50-fold with PBS as an activation reagent was used in the activation treatment of Example 17 (3).

The results of Examples 17 and 18 are shown together in Table 16. In Example 17, the relative value of the amount of luminescence for the anti-ALIX antibody with respect to the amount of luminescence for the mouse control antibody was higher in the cases where an activation reagent was added (Proteinase K: 6.36; HistoReveal: 7.28), compared to the case where the reagent was not added (PBS: 2.22). This result demonstrated that the addition of the activation reagent improves the relative value, and that the sensitivity could be improved with either enzyme treatment agent, that is, with either Proteinase K or HistoReveal, the latter containing trypsin. In particular, the amount of luminescence for the mouse control antibody decreased by the addition of the activation reagent, indicating that the activation treatment contributes to the suppression of non-specific adsorption.

In Example 18, when the activation treatment was carried out, improvement of the relative value could be achieved (1% formaldehyde: 7.57; 4% formaldehyde: 8.67) by increasing the amount of formaldehyde added, compared to the case where formaldehyde was not added (Example 17: 6.36). On the other hand, when the activation treatment was not carried out, the relative value only slightly increased by the increased amount of formaldehyde added (PBS: 2.22; 4% formaldehyde: 2.50). While formaldehyde is capable of cross-linking proteins to retain the proteins inside extracellular vesicle, the cross-linking causes masking of the recognition site for the antibody, leading to insufficient reactivity. It is assumed that, since cleavage of the cross-linking site by the activation treatment enables recovery of the binding capacity of the antibody to the antigen, the combination of the formaldehyde treatment and the activation treatment led to the increase in the amount of luminescence for the anti-ALIX antibody, resulting in the improvement of the detection sensitivity.

**[Table 16]**

| | Formaldehyde concentration [% (v/v)] | Membrane permeabilization treatment agent | Activation reagent | Amount of luminescence [104] | | Relative value |
|---|---|---|---|---|---|---|
| | | | | Anti-ALIX antibody | Mouse control antibody | |
| Example 17 | 0% | 40% (v/v) Ethanol | PBS | 19.79 | 8.92 | 2.22 |
| | 0% | | Proteinase K | 7.75 | 1.22 | 6.36 |
| | 0% | | HistoReveal | 12.82 | 1.76 | 7.28 |
| Example 18 | 1% | 40% (v/v) Ethanol | PBS | 10.14 | 4.18 | 2.43 |
| | 4% | | PBS | 10.15 | 4.06 | 2.50 |
| | 1% | | Proteinase K | 9.65 | 1.27 | 7.57 |
| | 4% | | Proteinase K | 14.03 | 1.62 | 8.67 |

| | | | | | | |
|---|---|---|---|---|---|---|
| Relative value: amount of luminescence for anti-ALIX antibody / amount of luminescence for mouse control antibody | | | | | | |

### Example 19

Color development of TMB was measured by the same method as in Example 1 except that the concentrated suspension of extracellular vesicles released from 293T cells was obtained from a different lot (lot number 7) of culture supernatant in Example 1 (1), that 0.5 µL of the concentrated suspension of extracellular vesicles was used in Example 1 (3), that 100 µL of an aqueous solution prepared by dissolving sodium deoxycholate at 0.075% (w/v) as a membrane permeabilization treatment agent in PBS was added in Example 1 (4), and that a plate reader manufactured by Corona Electric Co., Ltd. was used in Example 1 (9).

### Comparative Example 9

Color development of TMB was measured by the same method as in Example 19 except that PBS was used instead of the membrane permeabilization treatment agent in Example 19.

The results of Example 19 and Comparative Example 9 are shown in Table 17. Even when the volume of the extracellular vesicle suspension was 0.5 µL, the membrane permeabilization treatment with sodium deoxycholate resulted in a higher relative value of the absorbance for the anti-ALIX antibody with respect to the absorbance for the mouse control antibody (Example 19: 8.45), compared to the case with PBS (Comparative Example 9: 1.21). On the other hand, as described above, based on the results of Western blotting in Comparative Example 8, although the band derived from ALIX could be detected when the volume of the extracellular vesicle suspension was 5 µL, the band could not be detected when the volume was 0.5 µL.

Even when the amount of the extracellular vesicle was too small for the detection by the method of Patent Document 3, in which detection by Western blotting is carried out after allowing leakage of proteins from an extracellular vesicle, the present detection enabled highly sensitive detection of the protein in the extracellular vesicle by detecting the protein without allowing its leakage.

**[Table 17]**

| | Membrane permeabilization treatment agent | Absorbance | | Relative value |
|---|---|---|---|---|
| | | Anti-ALIX antibody | Mouse control antibody | |
| Example 19 | 0.075% (w/v) Sodium deoxycholate | 1.78 | 0.21 | 8.45 |
| Comparative Example 9 | PBS | 0.37 | 0.31 | 1.21 |

| | | | | |
|---|---|---|---|---|
| Relative value: absorbance for anti-ALIX antibody / absorbance for mouse control antibody | | | | |

### INDUSTRIAL APPLICABILITY

By the present invention, for example, the membrane permeabilization treatment of an extracellular vesicle for simply and accurately detecting a particular protein contained in the vesicles can be carried out. Further, by the present invention, for example, a particular protein contained in an extracellular vesicle can be simply and accurately detected. Thus, an internal protein in a particular extracellular vesicle can be detected among a plurality of types of extracellular vesicles. The method of the present invention is expected to be applicable not only to elucidation of physiological functions of an extracellular vesicle, but also to examination of diseases using a body fluid.

### DESCRIPTION OF SYMBOLS

100: Well
200: Magnet
300: Detector
10: Extracellular vesicle
11: Membrane-permeabilized extracellular vesicle
12: Labeled extracellular vesicle
20: Magnetic particle
30: Antibody immobilized on magnetic particle
40: Substrate
41: Product
50: Enzyme reaction

## Claims

1. A method of detecting a particular protein contained in an extracellular vesicle, the method comprising the steps of:
(A) capturing the extracellular vesicle using a carrier capable of binding to an extracellular-vesicle-specific marker present on the surface of the extracellular vesicle;
(B) carrying out membrane permeabilization treatment for the extracellular vesicle captured by the carrier, using a membrane permeabilization treatment agent; and
(C) introducing a reagent capable of detecting the particular protein contained in the extracellular vesicle, into the membrane-permeabilized extracellular vesicle, and
wherein the Step (B) is carried out such that the particular protein does not leak to the outside of the extracellular vesicle, and such that the reagent can be introduced into the extracellular vesicle.

2. The method according to claim 1, further comprising a step of removing an impurity after the Step (A) and/or after the Step (B).

3. The method according to claim 1 or 2, further comprising a step of detecting the particular protein using the introduced reagent after the Step (C).

4. The method according to any one of claims 1 to 3, wherein the membrane permeabilization treatment agent is a surfactant and/or an organic solvent.

5. The method according to claim 4, wherein the surfactant is an anionic surfactant, a cationic surfactant, or a nonionic surfactant.

6. The method according to claim 4 or 5, wherein the surfactant is deoxycholate, glycocholate, SDS, saponin, Triton X-100, or CTAB.

7. The method according to any one of claims 4 to 6, wherein the surfactant is any of the following (a) to (f):
(a) 0.05 to 0.5% (w/v) deoxycholate
(b) 0.01 to 1% (w/v) glycocholate
(c) 0.01 to 0.1% (w/v) SDS
(d) 0.1 to 2% (w/v) saponin
(e) 0.005 to 0.5% (w/v) Triton X-100
(f) 0.002 to 0.2% (w/v) CTAB.

8. The method according to any one of claims 4 to 7, wherein the surfactant is any of the following (a) to (c):
(a) 0.05 to 0.5% (w/v) deoxycholate
(b) 0.01 to 1% (w/v) glycocholate
(c) 0.01 to 0.1% (w/v) SDS.

9. The method according to any one of claims 4 to 8, wherein the organic solvent is ethanol and/or acetone.

10. The method according to any one of claims 4 to 9, wherein the organic solvent is the following (a) or (b):
(a) 20 to 60% (v/v) ethanol
(b) 20 to 70% (v/v) acetone.

11. The method according to any one of claims 1 to 10, wherein the carrier is a magnetic particle.

12. A membrane permeabilization treatment agent for an extracellular vesicle,
the agent comprising a surfactant and/or an organic solvent,
the agent being capable of allowing membrane permeabilization treatment of the extracellular vesicle such that a particular protein contained in the extracellular vesicle does not leak to the outside of the extracellular vesicle, and such that a reagent capable of detecting the particular protein contained in the extracellular vesicle can be introduced into the extracellular vesicle.

13. The membrane permeabilization treatment agent according to claim 12, wherein the surfactant is an anionic surfactant, a cationic surfactant, or a nonionic surfactant.

14. The membrane permeabilization treatment agent according to claim 12 or 13, wherein the surfactant is one or more components selected from the group consisting of deoxycholate, glycocholate, SDS, saponin, Triton X-100, and CTAB.

15. The membrane permeabilization treatment agent according to any one of claims 12 to 14, wherein the surfactant is any of the following (a) to (f):
(a) 0.05 to 0.5% (w/v) deoxycholate
(b) 0.01 to 1% (w/v) glycocholate
(c) 0.01 to 0.1% (w/v) SDS
(d) 0.1 to 2% (w/v) saponin
(e) 0.005 to 0.5% (w/v) Triton X-100
(f) 0.002 to 0.2% (w/v) CTAB.

16. The membrane permeabilization treatment agent according to any one of claims 12 to 15, wherein the surfactant is any of the following (a) to (c):
(a) 0.05 to 0.5% (w/v) deoxycholate
(b) 0.01 to 1% (w/v) glycocholate
(c) 0.01 to 0.1% (w/v) SDS.

17. The membrane permeabilization treatment agent according to any one of claims 12 to 16, wherein the organic solvent is ethanol and/or acetone.

18. The membrane permeabilization treatment agent according to any one of claims 12 to 17, wherein the organic solvent is the following (a) or (b):
(a) 20 to 60% (v/v) ethanol
(b) 20 to 70% (v/v) acetone.

19. A kit for detecting a particular protein contained in an extracellular vesicle, the kit comprising:
a magnetic particle capable of binding to an extracellular-vesicle-specific marker present on the surface of the extracellular vesicle;
the membrane permeabilization treatment agent according to any one of claims 12 to 18; and
a reagent capable of detecting the particular protein contained in the extracellular vesicle.
